# EUROPEAN PATENT SPECIFICATION

(11) **EP 0 585 396 B1**
(45) Date of publication and mention of the grant of the patent: **07.02.2001**
(21) Application number: 92913802.2
(22) Date of filing: 22.05.1992
(51) Int. Cl.: C12N 15/32, A01N 63/02, C12P 21/02, C12N 1/21, C12P 1/04

(54) **NOVEL BACILLUS THURINGIENSIS ISOLATES ACTIVE AGAINST HYMENOPTERAN PESTS AND GENES ENCODING HYMENOPTERAN-ACTIVE TOXINS**
GEGEN SCHÄDLINGE DER GATTUNG HAUTFLÜGLER AKTIVER BACILLUS THURINGIENSISISOLATE UND FÜR GEGEN HAUTFLÜGLER AKTIVE TOXINE KODIERENDE GENE
NOUVEAUX ISOLATS DE BACILLUS THURINGIENSIS PRESENTANT UNE ACTIVITE CONTRE LES HYMENOPTERES NUISIBLES ET GENES CODANT DES TOXINES AGISSANT CONTRE LES HYMENOPTERES

(30) Priority: 22.05.1991 US 703977; 25.11.1991 US 797645
(43) Date of publication of application: 09.03.1994
(60) Divisional application: 00114196.9
(73) Proprietor: MYCOGEN CORPORATION, San Diego, California 92121 (US)
(72) Inventor: PAYNE, Jewel, M., San Diego, CA 92126 (US); KENNEDY, M., Keith, Racine, WI 53402 (US); RANDALL, John, B., Racine, WI 53406 (US); MEIER, Henry, Racine, WI 53404 (US); UICK, Heidi, J., Racine, WI 53406 (US)
(74) Representative: Perry, Robert Edward
(86) International application number: US9204316
(87) International publication number: WO9220802

(56) References cited:
- EP-A- 0 200 344
- EP-A- 0 303 426
- EP-A- 0 462 721
- EP-A- 0 471 564
- BIOLOGICAL ABSTRACTS vol. 61 , 1 January 1976, Philadelphia, PA, US; abstract no. 1666, VANKOVA, J.E. ET AL. 'The control Monomorium pharaonis (Hymenoptera: Formicidae) with Bacillus thuringiensis'
- BIOLOGICAL ABSTRACTS vol. 60 , 1975, Philadelphia, PA, US; abstract no. 36681, WISNIEWSKI, J. 'Controlling Pharaoh ants in the zoo with Bacillus thuringiensis'

### SEQUENCE LISTING

(2) INFORMATION FOR SEQ ID NO:1:
   (i) SEQUENCE CHARACTERISTICS:
      (A) LENGTH: 3738 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus thuringiensis
      (C) INDIVIDUAL ISOLATE: PS86Q3
   (vii) IMMEDIATE SOURCE:
      (A) LIBRARY: Lambdagem (TM) - 11 LIBRARY
      (B) CLONE: 86Q3a
   (xi) SEQUENCE DESCRIPTION: SEQ ID NO:1
   ATGGCAACAA TTAATGAGTT GTATCCAGTT CCTTATAATG TGCTAGCTCA TCCAATTAAA     60
   GAAGTCGATG ATCCTTATTC TTGGTCAAAT TTATTAAAGG GTATACAAGA AGGTTGGGAA    120
   GAATGGGGAA AAACAGGACA AAAAAAACTT TTTGAAGACC ATCTTACGAT TGCATGGAAT    180
   CTTTATAAAA CAGGAAAATT AGATTATTTC GCTTTGACAA AAGCATCAAT ATCATTGATT    240
   GGATTTATTC CAGGGGCAGA AGCAGCAGTT CCCTTTATTA ATATGTTTGT AGACTTTGTT    300
   TGGCCTAAAT TATTTGGTGC GAATACAGAA GGAAAAGATC AACAGTTGTT TAATGCTATC    360
   ATGGATGCAG TTAATAAAAT GGTAGATAAT AAGTTCTTAA GTTATAATCT TAGTACACTT    420
   AATAAAACAA TTGAAGGACT TCAAGGTAAT TTAGGCCTAT TTCAAAATGC TATACAAGTA    480
   GCCATTTGTC AAGGCAGTAC ACCAGAAAGA GTAAATTTTG ATCAAAATTG TACACCATGT    540
   AATCCAAATC AACCTTGTAA AGATGATTTG GATAGAGTTG CTTCACGTTT TGATACGGCT    600
   AATTCTCAAT TCACACAGCA TTTACCAGAA TTTAAAAATC CTTGGTCGGA TGAAAACTCT    660
   ACTCAGGAAT TTAAAAGAAC ATCTGTTGAA TTAACTTTAC CAATGTATAC AACAGTAGCT 720
   ACGTTACATC TTTTATTATA TGAAGGATAT ATAGAATTTA TGACAAAATG GAATTTTCAC 780
   AATGAACAAT ATTTAAATAA TTTAAAGGTA GAATTACAAC AATTGATACA CTCATATTCA 840
   GAAACTGTTC GTACAAGTTT CCTTCAATTT TTACCTACCT TGAATAATCG TTCAAAATCA 900
   TCCGTAAATG CTTATAACCG TTATGTCCGC AATATGACTG TTAACTGTTT AGATATTGCT 960
   GCTACATGGC CTACATTTGA TACACATAAT TATCATCAAG GTGGTAAATT AGATTTAACT 1020
   CGTATTATTC TTTCAGATAC AGCAGGACCA ATAGAAGAAT ATACTACTGG CGACAAAACT 1080
   TCAGGACCTG AACATAGTAA CATTACACCA AATAATATTC TAGATACACC ATCTCCAACA 1140
   TATCAGCACT CATTTGTATC TGTTGATTCT ATTGTATATT CTAGAAAAGA ATTACAACAA 1200
   TTAGACATAG CTACTTATAG TACAAATAAT AGTAATAATT GTCACCCTTA TGGATTACGA 1260
   CTTTCATATA CAGATGGAAG CAGATATGAT TATGGAGATA ATCAACCTGA TTTTACTACT 1320
   TCCAATAACA ATTATTGTCA TAATAGCTAT ACTGCCCCTA TTACACTTGT GAATGCACGA 1380
   CATTTATATA ATGCAAAAGG CTCTTTACAA AATGTAGAAT CTTTAGTGGT TAGTACTGTA 1440
   AATGGTGGAA GTGGTTCATG CATTTGTGAT GCATGGATTA ATTATTTACG TCCTCCTCAA 1500
   ACAAGTAAAA ATGAATCACG TCCTGATCAA AAAATTAATG TTTTGTATCC AATAACAGAA 1560
   ACTGTAAATA AGGGGACTGG AGGAAATTTA GGAGTTATTT CTGCCTATGT TCCAATGGAA 1620
   CTTGTACCAG AAAACGTTAT TGGAGATGTT AATGCTGATA CTAAATTGCC ACTTACACAA 1680
   TTAAAGGGCT TTCCATTTGA AAAATATGGT TCTGAGTATA ATAATCGGGG TATCTCTCTT 1740
   GTTCGCGAAT GGATAAATGG TAACAATGCA GTTAAACTTT CTAATAGTCA ATCTGTTGGC 1800
   ATACAAATTA CGAATCAAAC CAAACAAAAA TATGAAATAC GTTGCCGTTA TGCGAGTAAA 1860
   GGAGATAATA ATGTTTATTT TAATGTGGAT TTAAGTGAAA ATCCATTTAG AAATTCCATT 1920
   TCTTTTGGAT CTACTGAAAG TTCTGTTGTA GGAGTACAAG GTGAAAATGG AAAGTATATA 1980
   TTGAAATCAA TCACAACGGT AGAAATACCT GCTGGAAGTT TCTATGTTCA TATAACAAAC 2040
   CAAGGTTCTT CAGATCTCTT TTTAGATCGT ATTGAGTTTG TTCCAAAAAT CCAATTCCAA 2100
   TTCTGTGATA ATAATAATCT TCACTGTGAT TGTAATAACC CTGTTGACAC CGATTGTACA 2160
   TTTTGTTGCG TTTGCACTAG TCTTACTGAT TGTGATTGTA ATAACCCTCG TGGCCTAGAT 2220
   TGTACGCTAT GTTGTCAGGT AGAAAATCAG CTACCTTCTT TTGTGACACT TACAGATTTA 2280
   CAAAATATTA CGACACAAGT AAATGCATTA GTTGCATCGA GCGAACATGA TACACTTGCA 2340
   ACAGACGTGA GTGATTATGA GATTGAAGAA GTTGTACTGA AAGTAGATGC ATTATCTGGT 2400
   GAAGTGTTTG GAAAAGAGAA AAAAGCATTG CGTAAATTGG TAAATCACAC AAAACGTTTA 2460
   AGCAAAGCGC GTAACCTCTT GATAGGAGGA AATTTTGATA ACTTGGATGC TTGGTACAGA 2520
   GGCCGAAATG TAGTAAACGT ATCTGATCAT GAACTATTTA AGAGTGATCA TGTATTATTG 2580
   CCACCACCAA CACTGTACTC ATCTTATATG TTCCAAAAAG TAGAGGAATC GAAATTAAAA 2640
   GCGAATACAC GTTATACTGT GTCTGGTTTT ATTGCACATG CAGAAGATTT AGAAATTGTT 2700
   GTGTCTCGTT ATGGGCAAGA AGTGAAGAAA GTGGTTCAAG TTCCATATGG AGAAGCATTC 2760
   CCATTGACAT CGAGGGGAGC GATTTGTTGC CCTCCACGTT CTACAAGTAA TGGAAAACCT 2820
   GCTGATCCAC ATTTCTTTAG TTACAGTATT GATGTGGGAA CATTAGATGT AGAAGCAAAC 2880
   CCTGGTATCG AATTGGGTCT TCGTATTGTA GAACGAACTG GAATGGCACG TGTAAGTAAT 2940
   TTAGAAATTC GTGAAGATCG TCCATTAAAG AAAAATGAAC TCCGCAATGT ACAACGTGCA 3000
   GCAAGAAATT GGAGAACAGC ATATGACCAA GAACGTGCAG AAGTAACGGC CTTGATTCAA 3060
   CCTGTATTAA ATCAAATCAA TGCGTTGTAT GAAAATGAAG ATTGGAATGG AGCAATTCGT 3120
   TCTGGAGTTT CTTATCATGA CTTAGAAGCA ATTGTTTTAC CAACATTACC AAAATTAAAT 3180
   CATTGGTTTA TGTCTGATAT GTTAGGGGAA CAAGGTTCCA TTTTAGCTCA ATTTCAAGAA 3240
   GCATTAGATC GTGCGTATAC GCAACTCGAA GAAAGTACAA TTCTGCATAA TGGTCATTTC 3300
   ACAACAGATG CAGCAAATTG GACGATAGAA GGCGATGCAC ATCATGCGAT ATTAGAAGAT    3360
   GGTAGACGCG TATTACGTCT TCCAGATTGG TCTTCTAGCG TTTCACAAAC CATTGAAATA    3420
   GAAAATTTTG ATCCAGATAA AGAATATCAG TTAGTTTTCC ATGCACAAGG AGAAGGAACG    3480
   GTCTCCCTTC AACATGGTGA AGAAGGAGAA TATGTGGAAA CACACCCGCA TAAGTCTGCG    3540
   AATTTTACAA CTTCACACCG TCAAGGAGTC ACATTTGAAA CAAATAAAGT AACAGTTGAA    3600
   ATTACCTCAG AAGATGGAGA ATTCCTAGTC GATCATATTG CTCTTGTGGA AGCTCCTCTT    3660
   CCTACAGATG ACCAAAGTTC AGATGGAAAT ACGACTTCCA ATACGAATAG CAATACAAGT    3720
   ATGAATAATA ATCAATAA                                                  3738
(2) INFORMATION FOR SEQ ID NO:2:
   (i) SEQUENCE CHARACTERISTICS:
      (A) LENGTH: 1245 amino acids
      (B) TYPE: amino acid
      (C) STRANDEDNESS: single
      (D) TOPOLOGY: linear
   (ii) MOLECULE TYPE: protein
   (iii) HYPOTHETICAL: YES
   (iv) ANTI-SENSE: NO
   (vi) ORIGINAL SOURCE:
      (A) ORGANISM: BACILLUS THURINGIENSIS
      (C) INDIVIDUAL ISOLATE: PS86Q3
   (vii) IMMEDIATE SOURCE:
      (A) LIBRARY: LAMBDAGEM (tm) - 11 library
      (B) CLONE: 86Q3A
   (xi) SEQUENCE DESCRIPTION: SEQ ID NO:2:
   Met Ala Thr Ile Asn Glu Leu Tyr Pro Val Pro Tyr Asn Val Leu Ala
   1               5                   10                  15
   His Pro Ile Lys Glu Val Asp Asp Pro Tyr Ser Trp Ser Asn Leu Leu
               20                  25                  30
   Lys Gly Ile Gln Glu Gly Trp Glu Glu Trp Gly Lys Thr Gly Gln Lys
           35                  40                  45
   Lys Leu Phe Glu Asp His Leu Thr Ile Ala Trp Asn Leu Tyr Lys Thr
       50                  55                  60
   Gly Lys Leu Asp Tyr Phe Ala Leu Thr Lys Ala Ser Ile Ser Leu Ile
   65                  70                  75                  80
   Gly Phe Ile Pro Gly Ala Glu Ala Ala Val Pro Phe Ile Asn Met Phe
                   85                  90                  95
   Val Asp Phe Val Trp Pro Lys Leu Phe Gly Ala Asn Thr Glu Gly Lys
               100                 105                 110
   Asp Gln Gln Leu Phe Asn Ala Ile Met Asp Ala Val Asn Lys Met Val
           115                 120                 125
   Asp Asn Lys Phe Leu Ser Tyr Asn Leu Ser Thr Leu Asn Lys Thr Ile
       130                 135                 140
   Glu Gly Leu Gln Gly Asn Leu Gly Leu Phe Gln Asn Ala Ile Gln Val
   145                 150                 155                 160
   Ala Ile Cys Gln Gly Ser Thr Pro Glu Arg Val Asn Phe Asp Gln Asn
                   165                 170                 175
   Cys Thr Pro Cys Asn Pro Asn Gln Pro Cys Lys Asp Asp Leu Asp Arg
               180                 185                 190
   Val Ala Ser Arg Phe Asp Thr Ala Asn Ser Gln Phe Thr Gln His Leu
           195                 200                 205
   Pro Glu Phe Lys Asn Pro Trp Ser Asp Glu Asn Ser Thr Gln Glu Phe
       210                 215                 220
   Lys Arg Thr Ser Val Glu Leu Thr Leu Pro Met Tyr Thr Thr Val Ala
   225                 230                 235                 240
   Thr Leu His Leu Leu Leu Tyr Glu Gly Tyr Ile Glu Phe Met Thr Lys
                   245                 250                 255
   Trp Asn Phe His Asn Glu Gln Tyr Leu Asn Asn Leu Lys Val Glu Leu
   260 265 270
   Gln Gln Leu Ile His Ser Tyr Ser Glu Thr Val Arg Thr Ser Phe Leu
   275 280 285
   Gln Phe Leu Pro Thr Leu Asn Asn Arg Ser Lys Ser Ser Val Asn Ala
   290 295 300
   Tyr Asn Arg Tyr Val Arg Asn Met Thr Val Asn Cys Leu Asp Ile Ala
   305 310 315 320
   Ala Thr Trp Pro Thr Phe Asp Thr His Asn Tyr His Gln Gly Gly Lys
   325 330 335
   Leu Asp Leu Thr Arg Ile Ile Leu Ser Asp Thr Ala Gly Pro Ile Glu
   340 345 350
   Glu Tyr Thr Thr Gly Asp Lys Thr Ser Gly Pro Glu His Ser Asn Ile
   355 360 365
   Thr Pro Asn Asn Ile Leu Asp Thr Pro Ser Pro Thr Tyr Gln His Ser
   370 375 380
   Phe Val Ser Val Asp Ser Ile Val Tyr Ser Arg Lys Glu Leu Gln Gln
   385 390 395 400
   Leu Asp Ile Ala Thr Tyr Ser Thr Asn Asn Ser Asn Asn Cys His Pro
   405 410 415
   Tyr Gly Leu Arg Leu Ser Tyr Thr Asp Gly Ser Arg Tyr Asp Tyr Gly
   420 425 430
   Asp Asn Gln Pro Asp Phe Thr Thr Ser Asn Asn Asn Tyr Cys His Asn
   435 440 445
   Ser Tyr Thr Ala Pro Ile Thr Leu Val Asn Ala Arg His Leu Tyr Asn
   450 455 460
   Ala Lys Gly Ser Leu Gln Asn Val Glu Ser Leu Val Val Ser Thr Val
   465 470 475 480
   Asn Gly Gly Ser Gly Ser Cys Ile Cys Asp Ala Trp Ile Asn Tyr Leu
   485 490 495
   Arg Pro Pro Gln Thr Ser Lys Asn Glu Ser Arg Pro Asp Gln Lys Ile
   500 505 510
   Asn Val Leu Tyr Pro Ile Thr Glu Thr Val Asn Lys Gly Thr Gly Gly
   515 520 525
   Asn Leu Gly Val Ile Ser Ala Tyr Val Pro Met Glu Leu Val Pro Glu
   530 535 540
   Asn Val Ile Gly Asp Val Asn Ala Asp Thr Lys Leu Pro Leu Thr Gln
   545 550 555 560
   Leu Lys Gly Phe Pro Phe Glu Lys Tyr Gly Ser Glu Tyr Asn Asn Arg
   565 570 575
   Gly Ile Ser Leu Val Arg Glu Trp Ile Asn Gly Asn Asn Ala Val Lys
   580 585 590
   Leu Ser Asn Ser Gln Ser Val Gly Ile Gln Ile Thr Asn Gln Thr Lys
   595 600 605
   Gln Lys Tyr Glu Ile Arg Cys Arg Tyr Ala Ser Lys Gly Asp Asn Asn
   610 615 620
   Val Tyr Phe Asn Val Asp Leu Ser Glu Asn Pro Phe Arg Asn Ser Ile
   625 630 635 640
   Ser Phe Gly Ser Thr Glu Ser Ser Val Val Gly Val Gln Gly Glu Asn
   645 650 655
   Gly Lys Tyr Ile Leu Lys Ser Ile Thr Thr Val Glu Ile Pro Ala Gly
   660 665 670
   Ser Phe Tyr Val His Ile Thr Asn Gln Gly Ser Ser Asp Leu Phe Leu
   675 680 685
   Asp Arg Ile Glu Phe Val Pro Lys Ile Gln Phe Gln Phe Cys Asp Asn
   690 695 700
   Asn Asn Leu His Cys Asp Cys Asn Asn Pro Val Asp Thr Asp Cys Thr
   705 710 715 720
   Phe Cys Cys Val Cys Thr Ser Leu Thr Asp Cys Asp Cys Asn Asn Pro
                   725                 730                 735
   Arg Gly Leu Asp Cys Thr Leu Cys Cys Gln Val Glu Asn Gln Leu Pro
               740                 745                 750
   Ser Phe Val Thr Leu Thr Asp Leu Gln Asn Ile Thr Thr Gln Val Asn
           755                 760                 765
   Ala Leu Val Ala Ser Ser Glu His Asp Thr Leu Ala Thr Asp Val Ser
       770                 775                 780
   Asp Tyr Glu Ile Glu Glu Val Val Leu Lys Val Asp Ala Leu Ser Gly
   785                 790                 795                 800
   Glu Val Phe Gly Lys Glu Lys Lys Ala Leu Arg Lys Leu Val Asn His
                   805                 810                 815
   Thr Lys Arg Leu Ser Lys Ala Arg Asn Leu Leu Ile Gly Gly Asn Phe
               820                 825                 830
   Asp Asn Leu Asp Ala Trp Tyr Arg Gly Arg Asn Val Val Asn Val Ser
           835                 840                 845
   Asp His Glu Leu Phe Lys Ser Asp His Val Leu Leu Pro Pro Pro Thr
       850                 855                 860
   Leu Tyr Ser Ser Tyr Met Phe Gln Lys Val Glu Glu Ser Lys Leu Lys
   865                 870                 875                 880
   Ala Asn Thr Arg Tyr Thr Val Ser Gly Phe Ile Ala His Ala Glu Asp
                   885                 890                 895
   Leu Glu Ile Val Val Ser Arg Tyr Gly Gln Glu Val Lys Lys Val Val
               900                 905                 910
   Gln Val Pro Tyr Gly Glu Ala Phe Pro Leu Thr Ser Arg Gly Ala Ile
           915                 920                 925
   Cys Cys Pro Pro Arg Ser Thr Ser Asn Gly Lys Pro Ala Asp Pro His
       930                 935                 940
   Phe Phe Ser Tyr Ser Ile Asp Val Gly Thr Leu Asp Val Glu Ala Asn
   945                 950                 955                 960
   Pro Gly Ile Glu Leu Gly Leu Arg Ile Val Glu Arg Thr Gly Met Ala
                   965                 970                 975
   Arg Val Ser Asn Leu Glu Ile Arg Glu Asp Arg Pro Leu Lys Lys Asn
               980                 985                 990
   Glu Leu Arg Asn Val Gln Arg Ala Ala Arg Asn Trp Arg Thr Ala Tyr
           995                 1000                1005
   Asp Gln Glu Arg Ala Glu Val Thr Ala Leu Ile Gln Pro Val Leu Asn
       1010                1015                1020
   Gln Ile Asn Ala Leu Tyr Glu Asn Glu Asp Trp Asn Gly Ala Ile Arg
   1025                1030                1035                1040
   Ser Gly Val Ser Tyr His Asp Leu Glu Ala Ile Val Leu Pro Thr Leu
                   1045                1050                1055
   Pro Lys Leu Asn His Trp Phe Met Ser Asp Met Leu Gly Glu Gln Gly
               1060                1065                1070
   Ser Ile Leu Ala Gln Phe Gln Glu Ala Leu Asp Arg Ala Tyr Thr Gln
           1075                1080                1085
   Leu Glu Glu Ser Thr Ile Leu His Asn Gly His Phe Thr Thr Asp Ala
       1090                1095                1100
   Ala Asn Trp Thr Ile Glu Gly Asp Ala His His Ala Ile Leu Glu Asp
   1105                1110                1115                1120
   Gly Arg Arg Val Leu Arg Leu Pro Asp Trp Ser Ser Ser Val Ser Gln
                   1125                1130                1135
   Thr Ile Glu Ile Glu Asn Phe Asp Pro Asp Lys Glu Tyr Gln Leu Val
               1140                1145                1150
   Phe His Ala Gln Gly Glu Gly Thr Val Ser Leu Gln His Gly Glu Glu
           1155                1160                1165
   Gly Glu Tyr Val Glu Thr His Pro His Lys Ser Ala Asn Phe Thr Thr
       1170                1175                1180
   Ser His Arg Gln Gly Val Thr Phe Glu Thr Asn Lys Val Thr Val Glu
   1185                1190                1195                1200
   Ile Thr Ser Glu Asp Gly Glu Phe Leu Val Asp His Ile Ala Leu Val
                   1205                1210                1215
   Glu Ala Pro Leu Pro Thr Asp Asp Gln Ser Ser Asp Gly Asn Thr Thr
               1220                1225                1230
   Ser Asn Thr Asn Ser Asn Thr Ser Met Asn Asn Asn Gln
           1235                1240                1245
(2) INFORMATION FOR SEQ ID NO:3:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:3:
   Arg Glu Trp Ile Asn Gly Ala Asn
   1               5
(2) INFORMATION FOR SEQ ID NO:4:
   (i) SEQUENCE CHARACTERISTICS:
      (A) LENGTH: 21 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:4:
   AGARTRKWTW AATGGWGCKM A 21
(2) INFORMATION FOR SEQ ID NO:5:
   (i) SEQUENCE CHARACTERISTICS:
      (A) LENGTH: 20 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:5:
   GARTGGWTAA ATGGTRMSAA 20
(2) INFORMATION FOR SEQ ID NO:6:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:6:
   Pro Thr Phe Asp Pro Asp Leu Tyr
   1               5
(2) INFORMATION FOR SEQ ID NO:7:
   (i) SEQUENCE CHARACTERISTICS:
      (A) LENGTH: 24 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:7:
   CCNACYTTTK ATCCAGATSW YTAT 24
(2) INFORMATION FOR SEQ ID NO:8:
   (i) SEQUENCE CHARACTERISTICS:
      (A) LENGTH: 24 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:8:
   CCWACWTTYG ATMCASATMW TTAT 24
(2) INFORMATION FOR SEQ ID NO:9:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:9:
   Met Ala Thr Ile Asn Glu Leu Tyr Pro Asn Val Pro Tyr Asn Val Leu
   1               5                   10                  15
(2) INFORMATION FOR SEQ ID NO:10:
   (i) SEQUENCE CHARACTERISTICS:
      (A) LENGTH: 37 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:10:
   AGACTGGATC CATGGCWACW ATWAATGAAT TATAYCC                    37
(2) INFORMATION FOR SEQ ID NO:11:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:11:
   Glu Ser Lys Leu Lys Pro Asn Thr Arg Tyr
   1               5                   10
(2) INFORMATION FOR SEQ ID NO:12:
   (i) SEQUENCE CHARACTERISTICS:
      (A) LENGTH: 29 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:12:
   TAACGTGTAT WCGSTTTTAA TTTWGAYTC                             29
(2) INFORMATION FOR SEQ ID NO:13:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:13:
   Tyr Ile Asp Lys Ile Glu Phe Ile Pro
   1               5
(2) INFORMATION FOR SEQ ID NO:14:
   (i) SEQUENCE CHARACTERISTICS:
      (A) LENGTH: 23 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:14:
   TGGAATAAAT TCAATTYKRT CWA 23
(2) INFORMATION FOR SEQ ID NO:15:
   (i) SEQUENCE CHARACTERISTICS:
      (A) LENGTH: 23 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:15:
   AGGAACAAAY TCAAKWCGRT CTA 23
(2) INFORMATION FOR SEQ ID NO:16:
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:17:
   Ile Thr Ser Glu Asp
   1               5
(2) INFORMATION FOR SEQ ID NO:18:
   (i) SEQUENCE CHARACTERISTICS:
      (A) LENGTH: 20 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:18:
   TCTCCATCTT CTGARGWAAT                                                20

## Description

### Background of the Invention

The development of biological control agents as alternatives to chemical insecticides for the control of important pest species is a subject of increasing interest. Concerns for the environment and exposure of man to harmful substances in air, food and water have stimulated legislation and restrictions regarding the use of chemical pesticides, particularly for pests found in the urban environment. Control of insect pests in urban areas is highly desirable but exposure to chemical pesticides in the household and from lawns and gardens is of great concern to the public. If given a choice, most people would use a non-toxic biological control rather than a toxic chemical to control insects in the urban environment. The problem is that very few biological alternatives to chemical insecticides are available for purchase and use by the public.

*Bacillus thuringiensis* (*B.t.*) produces an insect toxin designated as δ-endotoxin. It is synthesized by the *B.t.* sporulating cell. The toxin, upon being ingested in its crystalline form by susceptible insects, is transformed into biologically active moieties by the insect gut juice proteases. The primary target is insect cells of the gut epithelium, which are rapidly destroyed.

The reported activity spectrum of *B.t.* covers insect species within the order Lepidoptera, many of which are major pests in agriculture and forestry. The activity spectrum also includes the insect order Diptera, which includes mosquitos and black flies. See Couch, T.L. (1980) "Mosquito Pathogenicity of *Bacillus thuringiensis* var. *israelensis," Developments in Industrial Microbiology* 22:61-76; Beegle, C.C., (1978) "Use of Entomogenous Bacteria in Agroecosystems," *Developments in Industrial Microbiology* 20:97-104. Krieg, *et al.* (1983) *Z. ang. Ent.* 96:500-508, describe a *B.t.* isolate named *Bacillus thuringiensis* var. *tenebrionis,* which is reportedly active against two beetles in the order Coleoptera. These are the Colorado potato beetle, *Leptinotarsa decemlineata,* and *Agelastica alni.* In European Patent Application No. 0 202 739 there is disclosed a novel *B.t.* isolate active against Coleoptera and in European Patent Application No. 0 303 426 there is described a B.t. isolate active against nematodes. It is known as *B. thuringiensis* var. san diego (*B.t.s.d.*). U.S. Patent No. 4,966,765 discloses the coleopteran-active *Bacillus thuringiensis* isolate *B.t.* PS86B1.

Ants comprise a large group of insects (family Formicidae) from the taxonomic order, Hymenoptera. They are among the most common house pests. In many situations, ants are a nuisance pest. Foraging ants create problems with hygiene in hospitals and the food industry. Ants also create problems in agriculture. Damage can be caused by direct feeding on plants. Harvester and fire ants are commonly associated with this type of damage (Holldobler, B., E.O. Wilson [1990] *The Ants,* Belkap Press, Cambridge, Mass. 732 pp.) Some ants cause indirect damage by nurturing and protecting sap feeding insects such as mealybugs and aphids. Ants, particularly in the genus *Solenopsis* are capable of producing extremely painful stings to humans. It has been estimated that approximately 10,000 stings occur each year (Habermehl, G.G. [1981] *Venomous Animals and Their Toxins,* Springer-Verlag, NY, 195 pp.). The pharaoh ant (*Monomorium pharaonis*) is primarily an urban pest. However, this species can also be an agricultural pest and damage to corn has been noted (Ebeling, W. [1978] *Urban Entomology,* UC Press, Berkeley, Calif, 695 pp.).

Carpenter ants, *Camponotus* spp., are distributed throughout North America. Some of the more common and/or studied species include *C. modoc* in the Pacific northwest, *C. clarithorax* in southern California, and the black, red, and Florida carpenter ants, *C. pennsylvanicus*, *C. noveboracensis* and *C. abdominalis*, respectively, in the east (Ebeling, W. [1978] *Urban Entomology,* Univ. Calif.: Berkeley p. 209-213). Public concern over carpenter ants has been increasing due to the greater probability of structural infestations as suburban developments extend into the forest habitats of the ants.

Pestiferous species of carpenter ants may be considered nuisance pests because of their foraging activity inside homes. More significant damage occurs when carpenter ants extend their nests into sound wood. Nesting sites may be located in live and dead trees, sometimes resulting in damage to shade trees. Nests may also be established in walls and support beams of structures, or in voids within doors, walls, and furniture. Preference for moist or decaying wood has been reported, but nesting sites are not restricted to such areas. Carpenter ant populations develop relatively slowly with colonies of 300-2,000 workers being produced over a 2-year or longer period for various species. The presence of reproductives follows this slow development since their production has been reported only from well established colonies (Hansen, L.D., R.D. Akre [1985] Biology of carpenter ants in Washington state (Hymenoptera: Formicidae: *Camponotus*). Melanderia 43. 62 p.; Pricer, J.L. [1908] Biol. Bull. 14:177-218). Despite the slow colony growth, large colonies with satellite colonies have been found. Worker movement occurs between the main colony and the satellites, which serve as areas for further brood development and colony expansion (Hansen and Akre [1985], *supra*).

Current methods for controlling structural infestations of carpenter ants include sanitation of potential and current nest sites, minimizing access to structures (eg. preventing the contact of tree branches with a structure), and the application of insecticides to repel (perimeter spray barriers) and/or eliminate carpenter ants. The use of boric acid dust in dry, wall voids is reported to be effective for up to 20 years (Hansen and Akre, *supra*).

Recommendations for the chemical control of established structural infestations in the home are often accompanied with warnings of possible hazards to the applicator as well as children and pets. Alternative control methods such as effective biological control agents have not been found (Akre, R.D., L.D. Hansen, A.L. Antonelli [1989] Ext. Bull. Washington State Univ. Coop. Ext. Serv. 1989 rev. no. EB 0818, 6 pp.).

A need clearly exists for a safe, effective biological control agent for carpenter ants.

Pharaoh ants, *Monomorium pharaonis,* have been described as "... the most persistent and difficult of all our house-infesting ants to control or eradicate" (Smith, M.R. [1965] USDA-ARS Tech. Bull. No. 1326, 105 pp.). It is a tropical species which has extended its range to more temperate regions by establishing colonies in heated buildings. Pharaoh ants frequently infests buildings where food is prepared, and have been found to carry pathogenic organisms (Beatson, S.H. [1972] Lancet 1:425-427).

The difficulty in controlling pharaoh ants may be attributed to their inaccessible nesting sites, rapid population growth, and dispersion of colonies. Their small size allows establishment of colonies in any suitable location, including unusual places such as between books and in stored clothing. With multiple queen colonies, and the warm (30° C), humid (63-80% RH) conditions that favor pharaoh ants, large colonies can develop rapidly. Portions of these large colonies may disperse to form new colonies at any time, probably in response to overcrowding and unfavorable microenvironmental conditions. Unlike other ant species, pharaoh ants do not exhibit intercolony aggression. This permits the adoption of ants from other colonies and may further enhance the establishment of new colonies and reinfestations. Pharaoh ants also forage for food more than 35 m from the nest without distinct trail following, and thus make nests difficult to find and eradicate.

Control methods for pharaoh ants emphasize the use of insect growth regulators (IGR) or toxicants incorporated into baits. Properly implemented bait programs are effective, however it may take over a month to achieve control. Insecticide applications, while fast acting, usually do not eliminate colonies, and may be unacceptable in certain areas where toxic residues are a concern. In addition, insecticide applications are generally not compatible with bait programs.

A need exists for safe and effective biological control agents for pharaoh ants.

EP-A-0303426 discloses *B.t.* isolates PS-17, PS33F2 and PS63B.

EP-A-0462721 discloses *B.t.* toxins.

### Summary of the Invention

According to a first aspect of the present invention, a substantially pure toxin is a protein toxic to ants, of which the amino-acid sequence is greater than 75% the same as toxin 86Q3(a) as defined in SEQ ID NO. 2 or a part thereof having formicidal activity.

A second aspect of the present invention is a polynucleotide encoding a toxin of the invention. The polynucleotide can be transferred into a suitable host *via* a recombinant DNA vector.

### Detailed Disclosure of the Invention

One aspect of the subject invention is the discovery of a *Bacillus thuringiensis* isolate having activity against ants. The isolate (*B.t.* PS86Q3) has the characteristics, in its biologically pure form, given in Table 1.

**Table 1**

| Characteristics of *B.t.* PS86Q3 | |
|---|---|
| Colony morphology | large colony, dull surface, typical *B.t.* |
| | |
| Vegetative cell morphology | typical *B.t.* |
| | |
| Culture methods | typical for *B.t.* |
| | |
| Inclusions | long amorphic inclusion and a small inclusion, both of which remain with the spore after lysis |
| | |
| Approximate molecular weight of alkali/SDS-soluble polypeptides (kDa) | 155, 135, 98, 62, 58 |
| | |
| Serotype | new serotype (not H-1 through H-27) |
| | |
| Host range | Hymenoptera |

Toxins of the invention may be of the generic formula

Numbering is for convenience and approximate location only.

| Symbols used: | | | |
|---|---|---|---|
| A = ala | G = gly | M = met | S = ser |
| C = cys | H = his | N = asn | T = thr |
| D = asp | I = ile | P = pro | V = val |
| E = glu | K = lys | Q = gln | W = trp |
| F = phe | L = leu | R = arg | Y =tyr |
| | | | |
| K = K or R | | | |
| E = E or D | | | |
| L = L or I | | | |
| | | | |
| B = M, L, I, V, or F | | | |
| J = K, R, E, or D | | | |
| O = A or T | | | |
| U = N or Q | | | |
| Z = G or S | | | |
| X = any naturally occurring amino acid, except C. | | | |
| * = any naturally occurring amino acid. | | | |
| x = any naturally occurring amino acid, except C (or complete omission of any amino acids). | | | |

Where a stretch of wild-card amino acids is encountered (X(n) or x(n) where n>2), repetition of a given amino acid should be avoided. Similarly, P, C, E, D, K, or R utilization should be minimized.

Formicidal toxins according to the Generic Formula of the subject invention are specifically exemplified herein by the toxin encoded by the gene designated 86Q3(a). Since this toxin is merely exemplary of the toxins represented by the Generic Formula presented herein, it should be readily apparent that the subject invention further comprises equivalent toxins (and nucleotide sequences coding for equivalent toxins) having the same or similar biological activity as 86Q3(a). These equivalent toxins will have amino acid homology with 86Q3(a). This amino acid homology will be greater than 75%, and most preferably be greater than 90%. The amino acid homology will be highest in certain critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Table 2 provides a listing of examples of amino acids belonging to each class.

**Table 2**

| Class of Amino Acid | Examples of Amino Acids |
|---|---|
| Nonpolar | Ala, Val, Leu, lie, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the biological activity of the toxin. The information presented in the generic formulae of the subject invention provides clear guidance to the person skilled in this art in making various amino acid substitutions.

Further guidance for characterizing the formicidal toxins of the subject invention is provided in Tables 4 and 5, which demonstrate the relatedness among toxins within the formicidal toxins. These tables show a numeric score for the best matching alignment between two proteins that reflects: (1) positive scores for exact matches, (2) positive or negative scores reflecting the likelihood (or not) of one amino acid substituting for another in a related protein, and (3) negative scores for the introduction of gaps. A protein sequence aligned to itself will have the highest possible score―i.e., all exact matches and no gaps. However, an unrelated protein or a randomly generated sequence will typically have a low positive score. Related sequences have scores between the random background score and the perfect match score.

The sequence comparisons were made using the local homology algorithm of Smith and Waterman ([1981] *Advances in Applied Mathematics* 2:482-489), implemented as the program "Bestfit" in the GCG Sequence Analysis Software Package Version 7 April 1991. The sequences were compared with default parameter values (comparison table: Swgappep.Cmp, Gap weight:3.0, Length weight:0.1) except that gap limits of 250 residues were applied to each sequence compared. The program output value compared is referred to as the Quality score.

Tables 4 and 5 show the pairwise alignments between the indicated amino acids of the ant-active proteins and representatives of dipteran (CryIV; ISRH3 of Sen, K. *et al*. [1988] *Agric. Biol. Chem.* 52:873-878), lepidopteran and dipteran (CryIIA; CryB1 of Widner and Whiteley [1989] *J. Bacteriol*. 171:965-974), and lepidopteran (CryIA(c); Adang *et al.* [1981] *Gene* 36:289-300) proteins.

Table 3 shows which amino acids were compared from the proteins of interest.

**Table 3**

| Protein | Amino acids compared |
|---|---|
| 86Q3(a) | 1-697 |
| 63B | 1-692 |
| 33F2 | 1-618 |
| 17a | 1-677 |
| 17b | 1-678 |
| CryIV | 1-633 |
| CryIIA | 1-633 |
| CryIA(c) | 1-609 |

Table 4 shows the scores prior to adjustment for random sequence scores.

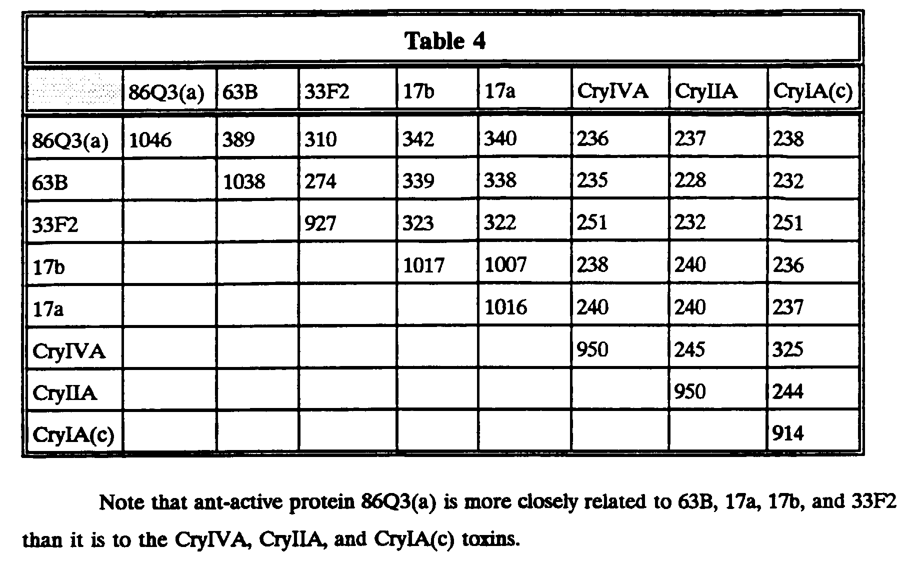

Table 5 shows the same analysis after subtraction of the average score of 50 alignments of random shuffles of the column sequences with the row sequences.

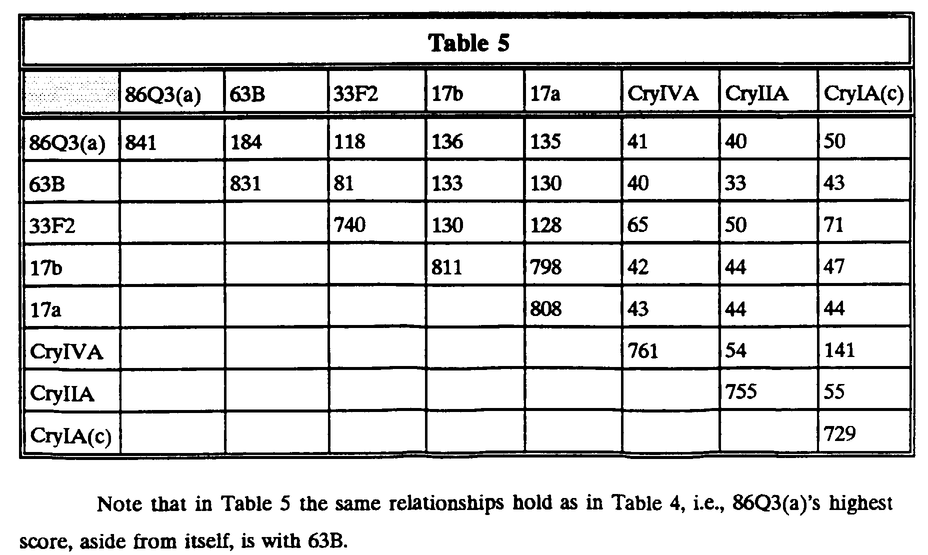

This degree of relatedness provides the basis for using common or similar sequence elements from the previously-described known genes to obtain related, but non-identical genes from an ant-active isolate.

Thus, certain toxins according to the subject invention can be defined as those which have formicidal activity and have an alignment value (according to the procedures of Table 5) greater than 100 with 86Q3(a). As used herein, the term "alignment value" refers to the scores obtained using the methods described above which were used to create the scores reported in Table 5.

The genes and toxins according to the subject invention include not only the full length sequences disclosed herein but also fragments of these sequences, or fusion proteins, which retain the characteristic formicidal activity of the sequences specifically exemplified herein.

It should be apparent to a person skilled in this art that genes coding for ant-active toxins can be identified and obtained through several means. The specific genes may be obtained from a culture depository as described below. These genes, or portions thereof, may be constructed synthetically, for example, by use of a gene machine. Variations of these genes may be readily constructed using standard techniques for making point mutations. Also, fragments of these genes can be made using commercially available exonucleases or endonucleases according to standard procedures. For example, enzymes such as *Bal*31 or site-directed mutagenesis can be used to systematically cut off nucleotides from the ends of these genes. Also, genes which code for active fragments may be obtained using a variety of other restriction enzymes. Proteases may be used to directly obtain active fragments of these toxins.

Equivalent toxins and/or genes encoding these equivalent toxins can also be located from *B.t.* isolates and/or DNA libraries using the teachings provided herein. There are a number of methods for obtaining the ant-active toxins of the instant invention which occur in nature. For example, antibodies to the ant-active toxins disclosed and claimed herein can be used to identify and isolate other toxins from a mixture of proteins. Specifically, antibodies may be raised to the portions of the ant-active toxins which are most constant and most distinct from other *B.t.* toxins. These antibodies can then be used to specifically identify equivalent toxins with the characteristic formicidal activity by immunoprecipitation, enzyme linked immunoassay (ELISA), or Western blotting. Antibodies to the toxins disclosed herein, or to equivalent toxins, or fragments of these toxins, can readily be prepared using standard procedures in this art. The genes coding for these toxins can then be obtained from the microorganism.

A further method for identifying the toxins and genes of the subject invention is through the use of oligonucleotide probes. These probes are nucleotide sequences having a detectable label. As is well known in the art, if the probe molecule and nucleic acid sample hybridize by forming a strong bond between the two molecules, it can be reasonably assumed that the probe and sample are essentially identical. The probe's detectable label provides a means for determining in a known manner whether hybridization has occurred. Such a probe analysis provides a rapid method for identifying formicidal endotoxin genes of the subject invention.

The nucleotide segments which are used as probes according to the invention can be synthesized by use of DNA synthesizers using standard procedures. In the use of the nucleotide segments as probes, the particular probe is labeled with any suitable label known to those skilled in the art, including radioactive and non-radioactive labels. Typical radioactive labels include ³²P, ¹²⁵I, ³⁵S, or the like. A probe labeled with a radioactive isotope can be constructed from a nucleotide sequence complementary to the DNA sample by a conventional nick translation reaction, using a DNase and DNA polymerase. The probe and sample can then be combined in a hybridization buffer solution and held at an appropriate temperature until annealing occurs. Thereafter, the membrane is washed free of extraneous materials, leaving the sample and bound probe molecules typically detected and quantified by autoradiography and/or liquid scintillation counting.

Non-radioactive labels include, for example, ligands such as biotin or thyroxine, as well as enzymes such as hydrolases or perixodases, or the various chemiluminescers such as luciferin, or fluorescent compounds like fluorescein and its derivatives. The probe may also be labeled at both ends with different types of labels for ease of separation, as, for example, by using an isotopic label at the end mentioned above and a biotin label at the other end.

Duplex formation and stability depend on substantial complementarity between the two strands of a hybrid, and, as noted above, a certain degree of mismatch can be tolerated. Therefore, the probes of the subject invention include mutations (both single and multiple), deletions, insertions of the described sequences, and combinations thereof, wherein said mutations, insertions and deletions permit formation of stable hybrids with the target polynucleotide of interest Mutations, insertions, and deletions can be produced in a given polynucleotide sequence in many ways, and these methods are known to an ordinarily skilled artisan. Other methods may become known in the future.

The known methods include, but are not limited to:
(1) synthesizing chemically or otherwise an artificial sequence which is a mutation, insertion or deletion of the known sequence;
(2) using a probe of the present invention to obtain via hybridization a new sequence or a mutation, insertion or deletion of the probe sequence; and
(3) mutating, inserting or deleting a test sequence *in vitro* or *in vivo.*

It is important to note that the mutational, insertional, and deletional variants generated from a given probe may be more or less efficient than the original probe. Notwithstanding such differences in efficiency, these variants are within the scope of the present invention.

Thus, mutational, insertional, and deletional variants of the disclosed test sequences can be readily prepared by methods which are well known to those skilled in the art. These variants can be used in the same manner as the instant probes so long as the variants have substantial sequence homology with the probes. As used herein, substantial sequence homology refers to homology which is sufficient to enable the variant to function in the same capacity as the original probe. Preferably, this homology is greater than 50%; more preferably, this homology is greater than 75%; and most preferably, this homology is greater than 90%. The degree of homology needed for the variant to function in its intended capacity will depend upon the intended use of the sequence. It is well within the skill of a person trained in this art to make mutational, insertional, and deletional mutations which are designed to improve the function of the sequence or otherwise provide a methodological advantage.

Specific nucleotide probes useful, according to the subject invention, in the rapid identification of ant-active genes are
(i) DNA coding for a peptide sequence whose single letter amino acid designation is "REWINGAN" (SEQ ID NO. 3) or variations thereof which embody point mutations according to the following: position 1, R or K; position 3, W or Y; position 4, I or L; position 7, A or N; position 8, N or Q; a specific example of such a probe is "AGA(A or G)T(G or A)(G or T)(A or T)T(A or T)AATGG(A or T)GC(G or T)(A or C)A" (SEQ ID NO. 4); another example of such a probe is "GA(A or G)TGG(A or T)TAAATGGT(A or G)(A or C)(G or C)AA" (SEQ ID NO. 5);
(ii) DNA coding for a peptide sequence whose single letter amino acid designation is "PTFDPDLY" (SEQ ID NO. 6) or variations thereof which embody point mutations according to the following: position 3, F or L; position 4, D or Y; position 5, P or T; position 6, D or H; position 7, L or H or D or N; a specific example of such a probe is "CC(A or T)AC(C or T)TTT(T or G)ATCCAGAT(C or G)(T or A)(T or C)TAT" (SEQ ID NO. 7); another example of such a probe is "CC(T or A)AC(T or A)TT(T or C)GAT(C or A)CA(G or C)AT(C or A)(T or A)TTAT" (SEQ ID NO. 8);
(iii) additional useful probes for detecting ant-active *B.t.* genes include "AGACTGGATC CATGGCWACW ATWAATGAAT TATAYCC" (SEQ ID NO. 10), "TAACGTGTAT WCGSTTTTAA TTTWGAYTC" (SEQ ID NO. 12), "TGGAATAAAT TCAATTYKRTCWA" (SEQ ID NO. 14), "AGGAACAAAY TCAAKWCGRT CTA" (SEQ ID NO. 15), and "TCTCCATCTT CTGARGWAAT" (SEQ ID NO. 18).

The potential variations in the probes listed is due, in part, to the redundancy of the genetic code. Because of the redundancy of the genetic code, i.e., more than one coding nucleotide triplet (codon) can be used for most of the amino acids used to make protein3. Therefore different nucleotide sequences can code for a particular amino acid. Thus, the amino acid sequences of the *B.t.* toxins and peptides can be prepared by equivalent nucleotide sequences encoding the same amino acid sequence of the protein or peptide. Accordingly, the subject invention includes such equivalent nucleotide sequences. Also, inverse or complement sequences are an aspect of the subject invention and can be readily used by a person skilled in this art. In addition it has been shown that proteins of identified structure and function may be constructed by changing the amino acid sequence if such changes do not alter the protein secondary structure (Kaiser, E.T., Kezdy, F.J. [1984] *Science* 223:249-255). Thus, the subject invention includes mutants of the amino acid sequence depicted herein which do not alter the protein secondary structure, or if the structure is altered, the biological activity is substantially retained. Further, the invention also includes mutants of organisms hosting all or part of a toxin encoding a gene of the invention. Such microbial mutants can be made by techniques well known to persons skilled in the art. For example, UV irradiation can be used to prepare mutants of host organisms. Likewise, such mutants may include asporogenous host cells which also can be prepared by procedures well known in the art.

The toxin genes or gene fragments exemplified according to the subject invention can be obtained from *B. thuringiensis* (*B.t.*) isolate designated PS86Q3. Subcultures of the *E. coli* host harboring the toxin genes of the invention were deposited in the permanent collection of the Northern Research Laboratory, U.S. Department of Agriculture, Peoria, Illinois, USA. The accession numbers are as follows:

| Culture | Repository No. | Deposit Date |
|---|---|---|
| *B.t.* PS86Q3 | NRRL B-18765 | February 6, 1991 |
| *E. coli* MR618(pMYC1647)(86Q3) | NRRL B-18970 | April 29, 1992 |

The *B.t.* isolates of the invention can be cultured using standard art media and fermentation techniques. Upon completion of the fermentation cycle, the bacteria can be harvested by first separating the *B.t.* spores and crystals from the fermentation broth by means well known in the art. The recovered *B.t.* spores and crystals can be formulated into a wettable powder, liquid concentrate, granules, or other formulations by the addition of surfactants, dispersants, inert carriers and other components to facilitate handling and application for particular target pests. These formulation and application procedures are all well known in the art.

Formulated products can be sprayed or applied as baits to control hymenopteran pests. When applied with a bait, the *B.t.* itself may be used, or another suitable host, as described herein, may be transformed with a *B.t.* gene and used to express toxins. A vegetable oil or other liquid substance can be added to a bait to make it more attractive to the pests. Various attractants, including pheromone compounds, are well known to those skilled in the art and can be used as a component of the bait. The bait and toxin or toxin-producing microbe can be used as part of a trap.

The *B.t.* cells of the invention can be treated prior to formulation to prolong the pesticidal activity when the cells are applied to the environment of a target pest Such treatment can be by chemical or physical means, or by a combination of chemical and/or physical means, so long as the technique does not deleteriously affect the properties of the pesticide, nor diminish the cellular capability in protecting the pesticide. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride; alcohols, such as isopropyl and ethanol; various histologic fixatives, such as Bouin's fixative and Helly's fixative (See: Humason, Gretchen. *L.,Animal Tissue Techniques,* W.H. Freeman and Company, 1967); or a combination of physical (heat) and chemical agents that prolong the activity of the toxin produced in the cell when the cell is applied to the environment of the target pest(s). Examples of physical means are short wavelength radiation such as gamma-radiation and X-radiation, freezing, UV irradiation, lyophilization, and the like.

Genes encoding toxins having activity against the target susceptible pests can be isolated from the *B.t.* isolate of the invention by use of well known procedures.

The toxin genes of the subject invention can be introduced into a wide variety of microbial hosts. Expression of the toxin gene results, directly or indirectly, in the intracellular production and maintenance of the pesticide. With suitable hosts, e.g., *Pseudomonas,* the microbes can be applied to the situs of hymenopteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the *B.t.* toxin.

Where the *B.t.* toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera *Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc,* and *Alcaligenes;* fungi, particularly yeast, e.g., genera *Saccharomyces, Cryptococcus, Kluyveromyces*, *Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus*, and *Azotobacter vinlandii*; and phytosphere yeast species such as *Rhodotorula rubra, R glutinis, R marina, R aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans.* Of particular interest are the pigmented microorganisms.

A wide variety of ways are available for introducing the *B.t.* gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA The initiation and translational termination region will involve stop codon(s), a terminator region, and optionally, a polyadenylation signal.

In the direction of transcription, namely in the 5' to 3' direction of the coding or sense sequence, the construct will involve the transcriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototropy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the *trp* gene, *lac* gene, *gal* gene, the lambda left and right promoters, the tac promoter, the naturally-occurring promoters associated with the toxin gene, where functional in the host See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host The replication system may be derived from the chromosome, an episomal element normally present in the host or a different host, or a replication system from a virus which is stable in the host A large number of plasmids are available, such as pBR322, pACYC184, RSF1010, pRO1614, and the like. See for example, Olson *et al*. (1982) *J. Bacteriol.* 150:6069; Bagdasarian *et al.* (1981) *Gene* 16:237; and U.S. Patent Nos. 4,356,270, 4,362,817, and 4,371,625.

The *B.t*. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as *Escherichia, Erwinia, Shigella, Salmonella,* and *Proteus;* Bacillaceae; Rhizobiceae, such as *Rhizobium;* Spirillaceae, such as photobacterium, *Zymomonas, Serratia, Aeromonas, Vibrio*, *Desulfovibrio, Spirillum;* Lactobacillaceae; Pseudomonadaceae, such as *Pseudomonas* and *Acetobacter*, Azotobacteraceae and Nitrobacteraceae. Among eukaryotes are fungi, such as Phycomycetes and Ascomycetes, which includes yeast, such as *Saccharomyces* and *Schizosaccharomyces;* and Basidiomycetes yeast, such as *Rhodotorula, Aureobasidium, Sporobolomyces,* and the like.

Characteristics of particular interest in selecting a host cell for purposes of production include ease of introducing the *B.t.* gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as *Rhodotorula* sp., *Aureobasidium* sp., *Saccharomyces* sp., and *Sporobolomyces* sp.; phylloplane organisms such as *Pseudomonas* sp., *Erwinia* sp. and *Flavobacterium* sp.; or such other organisms as *Escherichia, Lactobacillus* sp., *Bacillus* sp., *Streptomyces* sp., and the like. Specific organisms include *Pseudomonas aeruginosa, Pseudomonasfluorescens, Saccharomyces cerevisiae, Bacillus thuringiensis, Escherichia coli, Bacillus subtilis, Streptomyces lividans,* and the like.

The cell will usually be intact and be substantially in the proliferative form when treated, rather than in a spore form, although in some instances spores may be employed.

Treatment of the recombinant microbial cell can be done as disclosed *infra.* The treated cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the *B.t.* insecticidal gene may be grown in any convenient nutrient medium, where the DNA construct provides a selective advantage, providing for a selective medium so that substantially all or all of the cells retain the *B.t.* gene. These cells may then be harvested in accordance with conventional ways. Alternatively, the cells can be treated prior to harvesting.

The *B.t.* cells may be formulated in a variety of ways. They may be employed as wettable powders, baits, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the hymenopteran pest(s), e.g., plants, soil or water, by spraying, dusting, sprinkling, baits or the like.

Following are examples which illustrate procedures, including the best mode, for practicing the invention. These examples should not be construed as limiting. All percentages are by weight and all solvent mixture proportions are by volume unless otherwise noted.

### Example 1 - Culturing B.t. Isolates of the Invention

A subculture of a *B.t.* isolate can be used to inoculate the following medium, a peptone, glucose, salts medium.

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salts Solution | 5.0 ml/l |
| CaCl₂ Solution | 5.0 ml/l |

| Salts Solution (100 ml) | |
|---|---|
| M_{g}SO₄·7H₂O | 2.46 g |
| MnSO₄·H₂O | 0.04 g |
| ZnSO₄·7H₂O | 0.28 g |
| FeSO₄·7H₂O | 0.40 g |

| CaCl₂ Solution (100 ml) | |
|---|---|
| CaCl₂·2H₂O | 3.66 g |
| pH 7.2 | |

The salts solution and CaCl₂ solution are filter-sterilized and added to the autoclaved and cooked broth at the time of inoculation. Flasks are incubated at 30°C on a rotary shaker at 200 rpm for 64 hr.

### Example 2 - Purification of Protein and Amino Acid Sequencing

The *B.t.* isolate PS86Q3 was cultured as described in Example 1. The parasporal inclusion bodies were partially purified by sodium bromide (28-38%) isopycnic gradient centrifugation (Pfannenstiel, M.A., E.J. Ross, V.C. Kramer, K.W. Nickerson [1984] *FEMS Microbiol. Lett.* 21:39). The protein was bound to PVDF membranes (Millipore, Bedford, MA) by western blotting techniques (Towbin, H., T. Staehlelin, K. Gordon [1979] *Proc. Natl. Acad. Sci*. *USA* 76:4350) and the N-terminal amino acid sequence was determined by the standard Edman reaction with an automated gas-phase sequenator (Hunkapiller, M.W., R.M. Hewick, W.L. Dreyer, and L.E. Hood [1983] *Meth*. *Enzymol.* 91:399). The sequence obtained was:

From these sequence data oligonucleotide probes were designed by utilizing a codon frequency table assembled from available sequence data of other *B.t.* toxin genes. The probes were synthesized on an Applied Biosystems, Inc. DNA synthesis machine.

Protein purification and subsequent amino acid analysis of the N-terminal peptides listed above has led to the deduction of several oligonucleotide probes for the isolation of toxin genes from formicidal *B.t.* isolates. RFLP analysis of restricted total cellular DNA using radiolabeled oligonucleotide probes has elucidated different genes or gene fragments.

### Example 3 - Cloning of a Novel Toxin Gene from B.t. Isolate PS86Q3

Total cellular DNA was prepared from *Bacillus thuringiensis* (*B.t.*) cells grown to an optical density of 1.0 at 600 nm. The cells were recovered by centrifugation and protoplasts were prepared in lysis mix (300 mM sucrose, 25 mM Tris-HCl, 25 mM EDTA, pH = 8.0) containing lysozyme at a concentration of 20 mg/ml. The protoplasts were ruptured by addition of ten volumes of 0.1 M NaCl, 0.1% SDS, 0.1 M Tris-Cl, pH = 8.0. The cleared lysate was quickly frozen at -70°C and thawed to 37°C twice. The supernate was extracted twice with phenol:chloroform (1:1). The pellet was resuspended in 10 mM Tris-Cl, 1 mM EDTA, pH = 8.0 (TE), and RNase was added to a final concentration of 50 µg/ml. After incubation at 37°C for one hour, the solution was extracted once with phenol:chloroform (1:1) and then with TE-saturated chloroform. DNA was precipitated from the aqueous phase by the addition of one-tenth volume of 3M NaOAc and two volumes of ethanol. DNA was pelleted by centrifugation, washed with 70% ethanol, dried, and resuspended in TE.

Total cellular DNA from isolate PS86Q3 was used as template for polymerase chain reaction (PCR) analysis according to protocols furnished by Perkin Elmer Cetus. An oligonucleotide derived from the N-terminal amino acid sequence of the toxin protein was used as a 5' primer. The sequence of this oligonucleotide is:

An oligonucleotide coding for the amino acid sequence "ESKLKPNTRY" (SEQ ID NO. 11) can be used as the reverse 3' primer. The sequence of this oligonucleotide can be: "5'-TAACGTGTAT(A or T)CG(C or G)TTTTAATTT(T or A)GA(C or T)TC-3'" (SEQ ID NO. 12).

The reverse "YIDKIEFIP" (SEQ ID NO. 13) oligonucleotide was also used as a reverse 3' primer in conjunction with the above mentioned 5' primer. The sequence of the reverse primer can be: "5'-TGGAATAAATTCAATT(C or T)(T or G)(A or G)TC(T or A)A-3'" (SEQ ID NO. 14).

Amplification with the 5' primer and SEQ ID NO. 12 generates an approximately 2.3 kbp DNA fragment and an approximately 4.3 kbp DNA fragment. Amplification with the 5' primer and SEQ ID NO. 33 generates an approximate 1.8 kbp DNA fragment and an approximately 3.7 kbp DNA fragment. The approximately 2.3 kbp fragment was radiolabeled with ³²P and used as a hybridization probe to generate restriction fragment polymorphism (RFLP) patterns and to screen recombinant phage libraries.

A Southern blot of total cellular DNA digested with *EcoRV* was probed with the radiolabeled 2.3 kbp probe described above. The resultant RFLP includes 9.5 kbp, 6.4 kbp, and 4.5 kbp hybridizing fragments.

A gene library was constructed from PS86Q3 total cellular DNA partially digested with *Nde*II and size fractioned by gel electrophoresis. The 9-23 kb region of the gel was excised and the DNA was electroeluted and then concentrated using an Elutip-d ion exchange column (Schleicher and Schuel, Keene, NH). The isolated *Nde*II fragments were ligated into *Bam*HI-digested LambdaGEM-11 (PROMEGA). The packaged phage were plated on *E. coli* KW251 cells (PROMEGA) at a high titer and screened using the radiolabeled probe described above. Hybridizing plaques were purified and rescreened at a lower plaque density. Single isolated, purified plaques that hybridized with the probe were used to infect KW251 cells in liquid culture for preparation of phage for DNA isolation. DNA was isolated by standard procedures (Maniatis *et al., supra*). Preparative amounts of DNA were digested with *Sal*I (to release the inserted DNA from lambda sequences) and separated by electrophoresis on a 0.6% agarose-TAE gel. The large fragments were purified by ion exchange chromatography as above and ligated to *Sal*I-digested, dephosphorylated pHTBlueII (an *E. coli/B.t.* shuttle vector comprised of pBluescript S/K [Stratagene, San Diego, CA]) and the replication origin from a resident *B.t.* plasmid (Lereclus *et al.* [1989], *supra).* The ligation mix was introduced by transformation into competent *E. coli* NM522 cells (ATCC 47000) and plated on LB agar containing ampicillin, IPTG, and XGAL White colonies, with putative restriction fragment insertions in the (Beta)-galactosidase gene of pHTBlueII, were subjected to standard rapid plasmid purification procedures (Maniatis *et al., supra*). Plasmid DNA was analyzed by *Sal*I digestion and agarose gel electrophoresis. The desired plasmid construct, pMYC1647, contains an approximately 12 kb *Sal*I insert.

Plasmid pMYC1647 was introduced by electroporation into an acrystalliferous (Cry⁻) *B.t.*, HD-1 CryB (A.I. Aronson, Purdue University) host to yield MR515, a recombinant *B.t.* clone of 86Q3(a). Expression of an approximately 155 kDa protein was verified by SDS-PAGE. Spores and crystals were removed from broth cultures and were used for determination of toxicity to pharaoh ants.

### Example 4 - Activity of the B.t. Toxin Protein and Gene Product Against Ants

Broths were tested for the presence of β-exotoxin by a larval house fly bioassay (Campbell, D.P., Dieball, D.E., Bracket, J.M. [1987] "Rapid HPLC assay for the β-exotoxin of *Bacillus thuringiensis," J. Agric. Food Chem.* 35:156-158). Only isolates which tested free of β-exotoxin were used in the assays against ants.

A bait was made consisting of 10% *Bacillus thuringiensis* isolates of the invention and Crosse and Blackwell mint apple jelly. Approximately 100 ants were placed in each plastic test chamber replicate with the baits. Control experiments were performed with untreated mint apple jelly. Each test was replicated a minimum of 10 times. Mortality was assessed at 7, 14 and 21 days after introduction of the bait to the ants. Results are shown below:

**Table 6.**

| Toxicity of *B. thuringiensis* Isolates to the Pharaoh Ant (*Monomorium pharaonis*) | |
|---|---|
| *B.t.* Isolate | Percent Mortality |
| PS 86Q3 | 84 |
| Control | 11 |

### Example 5 - Activity Against Pharaoh Ants

Mint apple jelly containing 10% *B.t.* (100,000 ppm) was fed to 5 replicates of approximately 100 worker ants for 21 days. Total mortality (in %) over the test period is compared to control.

**Table 7.**

| Three week mortality (%) on pharaoh ant workers. | | |
|---|---|---|
| Sample | Rate ppm | Percent Mortality |
| MR515 | 100000 | 40.1 |
| 86Q3 | 100000 | 29.2 |
| MAJ | Blank | 25.0 |
| Control | Blank | 14.4 |
| MR515 = a recombinant *B.t.* clone of 86Q3(a) gene, 10% in MAJ (Example 3) | | |
| 86Q3 = spray dried powder of *B.t.* PS86Q3, 10% in MAJ | | |
| MAJ = Mint apple jelly, Crosse & Blackwell | | |
| Control = rearing diet of water, frozen flies, mealworms/honey agar | | |

**Table 8.**

| Three week mortality (%) on pharaoh ant workers. | | |
|---|---|---|
| Sample | Rate ppm | Percent Mortality |
| 86Q3 | 50000 | 99.6 |
| MAJ | Blank | 75.3 |
| Control | Blank | 39.0 |
| 86Q3 = 5% 86Q3 purified protein in MAJ | | |
| MAJ = Mint apple jelly, Crosse & Blackwell | | |
| Control = rearing diet of water, frozen flies, mealworms/honey agar | | |

### Example 6 - Cloning of Novel Ant-Active Genes Using Generic Oligonucleotide Primers

The formicidal gene of a new formicidal *B.t.* can be obtained from DNA of the strain by performing the standard polymerase chain reaction procedure as in Example 3 using the oligonucleotides of SEQ ID NO.14 or AGGAACAAAYTCAAKWCGRTCTA (SEQ ID NO. 15) as reverse primers and SEQ ID NO. 4, SEQ ID NO. 5, SEQ ID NO. 7, SEQ ID NO. 8 or SEQ ID NO. 10 as forward primers. The expected PCR fragments would be approximately 330 to 600 bp with either reverse primer and SEQ ID NO. 4 or SEQ ID NO. 5, 1000 to 1400 bp with either reverse primer and SEQ ID NO. 7 or SEQ ID NO. 8, and 1800 to 2100 bp with either reverse primer and the N-terminal primer SEQ ID NO. 10. Alternatively, a complement from the primer family described by SEQ ID NO. 4 and SEQ ID NO. 5 can be used as reverse primer with SEQ ID NO. 7, SEQ ID NO. 8 or SEQ ID NO. 10 as forward primers. The expected PCR fragments would be approximately 650 to 1000 bp with SEQ ID NO. 7 or SEQ ID NO. 8, and 1400 to 1800 bp for the N-terminal primer (SEQ ID NO. 10).

As another alternative, the reverse primer SEQ ID NO. 12 can be used with the N-terminal forward primer to yield fragments of approximately 2550-3100 bp; 1750-2150 bp with the forward primers SEQ ID NOS. 7 or 8; 850-1400 bp with SEQ ID NOS. 4 or 5; and 550-1050 bp with the forward primer TTTAGATCGT(A or C)TTGA(G or A)TTT(A or G)T(A or T)CC (SEQ ID NO. 16).

As yet another alternative, the ITSED (SEQ ID NO 17) reverse primer (TCTCCATCTTCTGA(G or A)G(T or A)AAT) (SEQ ID NO. 18) can be used with the N-terminal forward primer (SEQ ID NO. 10) to yield fragments of approximately 3550-4050 bp; 2600-3100 bp with forward primers SEQ ID NOS. 7 or 8, 1800-2400 bp with forward primers SEQ ID NOS. 4 or 5; and 1500-2050 bp with forward primer SEQ ID NO. 16.

Amplified DNA fragments of the indicated sizes can be radiolabeled and used as probes to clone the entire gene as in Example 3.

### Example 7 - Insertion of Toxin Gene Into Plants

One aspect of the subject invention is the transformation of plants with genes coding for a formicidal toxin. The transformed plants are resistant to attack by ants.

Genes coding for formicidal toxins, as disclosed herein, can be inserted into plant cells using a variety of techniques which are well known in the art. For example, a large number of cloning vectors comprising a replication system in *E. coli* and a marker that permits selection of the transformed cells are available for preparation for the insertion of foreign genes into higher plants. The vectors comprise, for example, pBR322, pUC series, M13mp series, pACYC184, etc. Accordingly, the sequence coding for the *B.t*. toxin can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted.

The use of T-DNA for the transformation of plant cells has been intensively researched and sufficiently described in EP 120 516; Hoekema (1985) In: *The Binary Plant Vector System,* Offset-durkkerij Kanters B.V., Alblasserdam, Chapter 5; Fraley *et al., Crit. Rev. Plant Sci* 4:1-46; and An *et al.* (1985) *EMBO J.* 4:277-287.

Once the inserted DNA has been integrated in the genome, it is relatively stable there and, as a rule, does not come out again. It normally contains a selection marker that confers on the transformed plant cells resistance to a biocide or an antibiotic, such as kanamycin, G 418, bleomycin, hygromycin, or chloramphenicol, *inter alia.* The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, or electroporation as well as other possible methods. If agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in agrobacteria. The intermediate vector can be transferred into *Agrobacterium tumefaciens* by means of a helper plasmid (conjugation). Binary vectors can replicate themselves both in *E. coli* and in agrobacteria. They comprise a selection marker gene and a linker or polylinker which are framed by the right and left T-DNA border regions. They can be transformed directly into agrobacteria (Holsters *et al.* [1978] *Mol. Gen. Genet.* 163:181-187). The agrobacterium used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

### Example 8 - Cloning of Novel B. thuringiensis Genes Into Insect Viruses

A number of viruses are known to infect insects. These viruses include, for example, baculoviruses and entomopoxviruses. In one embodiment of the subject invention, ant-active genes, as described herein, can be placed with the genome of the insect virus, thus enhancing the pathogenicity of the virus. Methods for constructing insect viruses which comprise *B.t.* toxin genes are well known and readily practiced by those skilled in the art. These procedures are described, for example, in Merryweather *et al.* (Merryweather, A.T., U. Weyer, M.P.G. Harris, M. Hirst, T. Booth, R.D. Possee (1990) *J. Gen. Virol.* 71:1535-1544) and Martens et aL (Martens, J.W.M., G. Honee, D. Zuidema, J.W.M. van Lent, B. Visser, J.M. Vlak (1990) *Appl. Environmental Microbiol.* 56(9):2764-2770).

## Claims

1. A substantially pure toxin which is a protein toxic to ants, of which the amino-acid sequence is greater than 75% the same as toxin 86Q3(a) as defined in SEQ ID NO. 2 or a part thereof having formicidal activity.

2. The toxin according to claim 1, which is immunoreactive with an antibody which immunoreacts with toxin 86Q3(a).

3. The toxin according to claim 1, which comprises all or part of SEQ ID NO. 2.

4. The toxin according to claim 1, which comprises SEQ ID NO. 2.

5. A polynucleotide encoding a toxin as defined in any of claims 1 to 4.

6. The polynucleotide according to claim 5, which hybridises with DNA which codes for all or part of toxin 86Q3(a) having formicidal activity.

7. The polynucleotide according to claim 5, which comprises all or part of SEQ ID NO. 1 encoding a toxin having formicidal activity.

8. The polynucleotide according to claim 5, which comprises SEQ ID NO. 1.

9. A transformed host comprising a nucleotide sequence which codes for, and optionally also expresses, a toxin as defined in any of claims 1 to 4.

10. A host according to claim 9, which is *Bacillus thuringiensis.*

11. Substantially intact cells which express a toxin according to any of claims 1 to 4.

12. A process for controlling ants, which comprises contacting the ants with a toxin according to any of claims 1 to 4, a host according to claim 9 or claim 10, or cells according to claim 11.

## Patentansprüche

1. Im wesentlichen reines Toxin, welches ein für Ameisen toxisches Protein ist, dessen Aminosäuresequenz zu mehr als 75% mit dem Toxin 86Q3 (a) wie in SEQ-ID-Nr. 2 definiert oder einem Teil davon mit ameisentötender Aktivität identisch ist.

2. Toxin nach Anspruch 1, welches mit einem Antikörper, der mit Toxin 86Q3 (a) eine Immunreaktion zeigt, eine Immunreaktion zeigen kann.

3. Toxin nach Anspruch 1, welches SEQ-ID-Nr. 2 ganz oder teilweise umfaßt.

4. Toxin nach Anspruch 1, welches SEQ-ID-Nr. 2 umfaßt.

5. Polynukleotid, kodierend für ein Toxin wie in irgendeinem der Ansprüche 1 bis 4 definiert.

6. Polynukleotid nach Anspruch 5, welches mit DNA hybridisiert, die für das gesamte Toxin 86Q3 (a) oder einen Teil davon mit ameisentötender Aktivität kodiert.

7. Polynukleotid nach Anspruch 5, welches SEQ-ID-Nr. 1 ganz oder einen für ein Toxin mit ameisentötender Aktivität kodierenden Teil davon umfaßt.

8. Polynukleotid nach Anspruch 5, welches SEQ-ID-Nr. 1 umfaßt.

9. Transformierter Wirt, umfassend eine Nukleotidsequenz, die ein Toxin wie in irgendeinem der Ansprüche 1 bis 4 definiert kodiert und gegebenenfalls auch exprimiert.

10. Wirt nach Anspruch 9, welcher *Bacillus thuringiensis* ist.

11. Im wesentlichen intakte Zellen, welche ein Toxin nach irgendeinem der Ansprüche 1 bis 4 exprimieren.

12. Verfahren zur Bekämpfung von Ameisen, welches das Kontaktieren der Ameisen mit einem Toxin nach irgendeinem der Ansprüche 1 bis 4, einem Wirt nach Anspruch 9 oder Anspruch 10 oder Zellen nach Anspruch 11 umfaßt.

## Revendications

1. Toxine sensiblement pure qui est une protéine toxique pour les fourmis, dont la séquence d'acides aminés est identique à plus de 75 % à la toxine 86Q3 (a) définie dans SEQ ID NO. 2 ou une partie de celle-ci ayant une activité formicide.

2. Toxine selon la revendication 1, qui est immunoréactive avec un anticorps qui immunoréagit avec la toxine 86Q3 (a).

3. Toxine selon la revendication 1, qui comprend tout ou partie de SEQ ID NO. 2.

4. Toxine selon la revendication 1, qui comprend SEQ ID NO.2.

5. Polynucléotide codant une toxine selon l'une quelconque des revendications 1 à 4.

6. Polynucléotide selon la revendication 5 qui s'hybride avec un ADN qui code tout ou partie de la toxine 86Q3 (a) ayant une activité formicide.

7. Polynucléotide selon la revendication 5, qui comprend tout ou partie de SEQ ID NO. 1 codant une toxine ayant une activité formicide.

8. Polynucléotide selon la revendication 5, qui comprend SEQ ID NO.1.

9. Hôte transformé comprenant une séquence nucléotidique qui code, et éventuellement aussi exprime, une toxine selon l'une quelconque des revendications 1 à 4.

10. Hôte selon la revendication 9 qui est *Bacillus thuringiensis.*

11. Cellules sensiblement intactes qui expriment une toxine selon l'une quelconque des revendications 1 à 4.

12. Procédé de lutte contre les fourmis qui comprend la mise en contact des fourmis avec une toxine selon l'une quelconque des revendications 1 à 4, un hôte selon la revendication 9 ou la revendication 10 ou des cellules selon la revendication 11.
